# EUROPÄISCHE PATENTANMELDUNG

(11) **EP 1 707 964 A2**
(43) Veröffentlichungstag der Anmeldung: **04.10.2006**
(21) Anmeldenummer: 06002407.2
(22) Anmeldetag: 07.02.2006
(51) Int. Cl.: G01N 33/68

(54) **Verfahren zur Untersuchung und Bestimmung von proteinhaltigen Strukturen**

(30) Priorität: 10.03.2005 DE 102005010852; 24.03.2005 DE 102005013623
(71) Anmelder: Schallmey, Wolfgang, 48231 Warendorf (DE)
(72) Erfinder: Schallmey, Wolfgang, 48231 Warendorf (DE)
(74) Vertreter: Habbel, Hans-Georg

(57) **Zusammenfassung**

Die Erfindung betrifft ein. Verfahren zur Untersuchtung und Bestimmung von proteinhaltigen Strukturen im Hinblick auf das Krankheitsbild der atopischen Erkrankungen unter Verwendung des Farbstoffes Pyrogallol-Rot in der Färbelösung mit dem Handelsnamen **"redprot"** als Proteindetektor, wobei die mit einer **"redprot"** bedeckte Untersuchungsprobe auf ein durchsichtiges Trägermaterial bzw. auf ein reflektierendes aufgebracht und hinsichtlich Struktur und Färbung proteinhaltiger Strukturen, d. h. biologischen Materials, untersucht wird.

## Beschreibung

Die Erfindung bezieht sich auf ein Verfahren zur Untersuchung und Bestimmung von proteinhaltigen Strukturen gemäß dem Oberbegriff des Hauptanspruches.

Proteinhaltige Strukturen, wie beispielsweise Milben, Milbenexkremente, Pollen, Schimmelpilze, Hautschuppen und pflanzliche Fragmente von Gräsern und Bäumen befinden sich in großem Umfang in der Luft und können zu schwerwiegenden allergischen Reaktionen bei entsprechend empfindlichen Menschen führen.

Aus der EP 0 815 451 B1 ist ein Verfahren zur Untersuchung von Hausstaub im Hinblick auf ein allergische Reaktionen auslösendes Potential bekanntgeworden, wobei dieses Verfahren sich dadurch kennzeichnet, daß der Hausstaub mit einem Proteindetektor versetzt wird und anschließend ein größenordnungsmäßiges Feststellen von proteinhaltigen Bestandteilen erfolgt. Als Proteindetektor wird eine Farblösung eingesetzt, die den Farbstoff enthält, wie er als Pyro-gallol-Rot bekannt geworden ist. Bei dem Farbstoff Pyrogallol-Rot handelt es sich um Pyrogallolsulfophtalein (C₁₉H₁₂O₈S mit dem Molekulargewicht 400,36 dalton).

Die genannte Literaturstelle bezieht sich auch auf die Verwendung des Farbstoffes Pyrogallol-Rot zur Abschätzung eines allergische Reaktion auslösenden Potentials von Hausstaub.

Bei diesem bekannten Verfahren und bei dieser bekannten Verwendung wird so vorgegangen, daß eine entsprechende Stammlösung auf ein muldenartig geformtes Filterstück aus Polypropylen aufgegeben wird. Dieses Filterstück wird gut durchtränkt. Je nach Umgebungstemperatur und Menge und Art des gebundenen Feinstaubes färbt sich das Staubmaterial im Filter je nach Proteinbelastung nach maximal 5 Minuten blau-violett.

Mit diesem bekannten Verfahren kann also lediglich festgestellt werden, ob proteinhaltige Strukturen in der Probe enthalten sind.

Ausgehend von diesem Stand der Technik ist eine technische Problematik darin zu sehen, daß bei diesem bekannten Verfahren keine differenzierte Analyse möglich ist, d. h. es kann nach dem bekannten Verfahren nicht differenziert festgestellt werden, ob die proteinhaltige Struktur, die in der Probe enthalten ist, durch Milben, durch Pollen, durch Pilze oder sonstige proteinhaltige Materialien hervorgerufen wird, und in welchem Umfang die proteinhaltigen Strukturen vorliegen.

Der Erfindung liegt daher die Aufgabe zugrunde, ein Verfahren zu schaffen, mit dem eine differenzierte Analyse erfolgen kann, und zur Lösung dieser Aufgabe wird ein Verfahren vorgeschlagen, wie es im Hauptanspruch und im selbständigen Anspruch 2 definiert ist.

Durch die neu vorgeschlagene Verfahrensweise wird ein innovativer Beitrag zur wichtigen praktischen Anwendung vorgeschlagen, die
1. die Identifikation der Pollen und Pilzsporen in routinemäßigen aerobiolo gischen Analysen und
2. die Verifizierung der Korrelation von biologisch und allergener Aktivität mit dem möglichen Krankheitsbild der atopischen Erkrankungen ermöglicht.
3. Insbesondere gibt die erfindungsgemäße Verfahrensweise die Möglichkeit, biologisch aktive proteinhaltige Strukturen zu er mitteln und von biologisch toten proteinhaltigen Strukturen zu unterscheiden.

Dieser neue Aspekt, der auf Ergebnissen aus der mikrospektroskopischen Analyse und der "redprot"-Färbung von Materialien basiert, das mit Pollenfallen oder auf entsprechende Weise gesammelt wurde, schafft also die Möglichkeit, differenziert festzustellen, ob die in der Probe enthaltene proteinhaltige Struktur von Milben, Milbenexkremente, Schimmelpilze, Pollen oder sonstige pflanzliche Partikel oder Hautschuppen herrührt.

Die Durchführung des erfindungsgemäßen Verfahrens erfolgt vorzugsweise wie folgt:

Das auf einem Objektträger (48 x 12 mm) gesammelte Material, d. h. also die Untersuchungsprobe, wird mit 0,5 ml der "redprot"-Lösung, i.e. 0,02 % Lösung Pyrogallol-rot in 30 % isopropanol mit pH 2,0, für 3 Minuten bedeckt.

Nach dem Färbevorgang wird die restliche Farblösung sorgfältig entfernt und die Probe etwa 2 Minuten lang bei Zimmertemperatur getrocknet.

Für die Versiegelung und Konservierung der Probe wird ein Medium von Glycerin-Gelatine-Phenol nach Lanzoni verwendet, das vorher bei 46° C verflüssigt wurde.

Zum Schluß wird die Probe für die Analyse unter dem optischen Mikroskop mit dem Deckglas bedeckt.

Das Aufbringen der flüssigen Färbelösung auf dem Trägermaterial mit Probe kann durch Tauchen, Besprühen, Elektrosprühen erfolgen und die Probe wird auf dem Träger mit implantierten Farbpartikeln gesammelt.

Die Auswertung erfolgt mittels Bildanalyse, d. h. der CCD-Kamera oder mittels einer Spektralanalyse mit Spektrofotometer.

Bei einer anderen Verfahrensweise werden 2 ml bei 46° C verflüssigtes Gelatine-Glycerin-Phenol nach LANZONI mit 1 ml Pyrogallol-Lösung vermischt, d.h. in einem Verhältnis von 3 : 1. Der pH-Wert der gewonnenen Lösung sollte sich zwischen 3.0 bis 3.5 bewegen.
Die Farblösung sollte sofort verwendet werden und die Dauer der Färbung schwankt zwischen 4 und 6 Stunden.

## Patentansprüche

1. Verfahren zur Untersuchung und Bestimmung von proteinhaltigen Strukturen im Hinblick auf das Krankheitsbild der atopischen Erkrankungen unter Verwendung des Farbstoffes Pyrogallol-Rot in der Färbelösung mit dem Handelsnamen "**redprot**" als Proteindetektor, **dadurch gekennzeichnet, daß** die mit einer "**redprot**" bedeckte Untersuchungsprobe auf ein durchsichtiges Trägermaterial aufgebracht und hinsichtlich Struktur und Färbung proteinhaltiger Strukturen, d. h. biologischen Materials, untersucht wird.

2. Verfahren zur Untersuchung und Bestimmung von proteinhaltigen Strukturen im Hinblick auf das Krankheitsbild der atopischen Erkrankungen unter Verwendung der Pyrogallol-Rot-haltigen Farblösung **"redprot"** als Proteindetektor, **dadurch gekennzeichnet, daß** die mit einer **"redprot"** bedeckte Untersuchungsprobe auf ein reflektierendes Trägermaterial aufgebracht und hinsichtlich Struktur und Farbtönung des proteinhaltigen biologischen Materials untersucht wird.

3. Verfahren nach Anspruch 1 oder 2, **dadurch gekennzeichnet, daß** das ein Quadratzentimeter (cm²) des gesammelten Materials bzw. der Untersuchungsprobe mit ca. 0,2 ml der "**redprot**"-Lösung [0,02 % Lösung Pyrogallol-Rot in 30 % Isopropanol mit pH 2,0] für drei Minuten bedeckt wird, nach dem Färbevorgang die restliche Farblösung sorgfältig entfernt und die Probe etwa zwei Minuten lang bei Zimmertemperatur getrocknet wird,
für die Versiegelung und Konservierung der Probe ein farblosem Medium von Glycerin-Gelatine-Phenol nach Rezeptur von Lanzoni verwendet wird, das vorher bei 46° C verflüssigt wurde und
anschließend die Probe für die Analyse unter dem optischen Mikroskop mit einem Deckglas bedeckt wurde.

4. Verfahren nach Anspruch 1 oder 2, **dadurch gekennzeichnet, daß** im Verhältnis 3 : 1 farbloses, bei 46° C verflüssigtes Gelatine-Glycerin-Phenol nach LANZONI (2 ml) mit **"redprot"** Lösung (1 ml) vermischt wird,
der pH Wert der gewonnenen Lösung zwischen 3,0-3,5 eingestellt wird und
die Farblösung sofort verwendet wird. Die Dauer der färbung schwankt zwischen 4 und 6 Stunden.

5. Verfahren nach einem oder mehreren der vorhergehenden Ansprüche 1 bis 4, **dadurch gekennzeichnet, daß** die Auswertung und Bildanalyse mittels einer CCD- oder VideoKamera erfolgt.

6. Verfahren nach einem oder mehreren der vorhergehenden Ansprüche 1 bis 4, **dadurch gekennzeichnet, daß** die Auswertung der Probe mittels einer Spektralanalyse im Spektrofotometer erfolgt.
